# EUROPEAN PATENT SPECIFICATION

(11) **EP 0 398 890 B1**
(45) Date of publication and mention of the grant of the patent: **08.02.1995**
(21) Application number: 89900198.6
(22) Date of filing: 30.11.1988
(51) Int. Cl.: A61B 5/20, A61F 5/451, G01F 19/00

(54) **AN APPARATUS FOR THE COLLECTION AND MEASUREMENT OF BODY LIQUIDS**
ANORDNUNG ZUR SAMMLUNG UND MESSUNG VON KÖRPERFLÜSSIGKEITEN
APPAREIL SERVANT A RECUEILLIR ET A MESURER DES LIQUIDES CORPORELS

(30) Priority: 30.11.1987 DK 6282/87
(43) Date of publication of application: 28.11.1990
(73) Proprietor: UNO PLAST A/S, DK-3390 Hundested (DK)
(72) Inventor: SVENDSEN, Gunnar, DK-4040 Jyllinge (DK)
(74) Representative: Kyed, Iver
(86) International application number: DK8800196
(87) International publication number: WO8905119

(56) References cited:
- SE-B- 447 336
- US-A- 4 579 126
- US-A- 4 625 734

## Description

The present invention relates to an apparatus for the collection and measurement of body liquids, which apparatus comprises a measuring vessel divided into at least two measuring chambers and having at its upper end a liquid inlet and at its lower end a liquid outlet provided with a valve and a liquid collection bag connected to the liquid outlet and suspended from the measuring vessel.

Apparatus of the kind defined above are used e.g. in hospitals for monitoring the urine discharge from bedridden patients and in particular from patients with catheters inserted into their urine bladders.

The catheter is connected to the liquid inlet of the measuring vessel and the measuring vessel and the urine bag connected thereto are typically suspended from the side of the patient's bed. The urine bag may for instance be suspended from two hooks mounted on carrier arms extending from the measuring vessel. When the patient is transported in his bed from one place to another or is moved into another bed the measuring vessel and the urine bag are normally positioned in an inclined position between the patient's legs.

US patent specification No. 4,625,734 discloses an urine meter comprising a flexible drainage bag which may be suspended vertically from a hospital bed and a relatively rigid urine metering collection chamber. An urine drain tube extends into the collection chamber to form a burette which at its upper end communicates with the main body of the metering collection chamber. The main body in turn communicates at its upper end with the drainage bag. However, the metering collection chamber can only be emptied by raising it to a horizontal position whereby urine flows into the flexible bag from which it can be discharged through a valve provided at the lower end of said bag.

An apparatus as described above is known from EP patent publication No. 0,008,450. The known apparatus comprises a measuring vessel divided into four measuring chambers by four substantially vertical separating walls and an overflow chamber which is directly connected to the urine bag. The four measuring chambers may be selectively connected to the urine bag by means of a valve thus permitting selective transfer of the contents of the measuring chambers into the urine bag. The separating walls between said chambers are designed so as to permit the urine to overflow into the second chamber once the first is filled, and so on.

If the measuring vessel is positioned in an inclined position e.g. in connection with the above discussed movement of a bedridden patient there is a risk involved in using the prior art apparatus in that urine may unintentionally flow from one measuring chamber to another and optionally into the overflow chamber thus preventing accurate reading of the amount of urine discharged within a certain period of time.

It is the object of the present invention to avoid such unintentional overflow of body liquid from one measuring chamber to another or to the liquid collection bag when the apparatus is placed in an inclined position.

It is a further object of the invention to provide an apparatus of a simpler construction and operation than those of the known apparatus.

These objects are obtained with the apparatus according to the invention, said apparatus being characterized in that the liquid outlet has the shape of a valve seat for a hollow valve body which is vertically displaceable within the measuring vessel, the interior of the valve body being divided into (a) a reception chamber connected to the liquid inlet and having at the lower end of the valve body at least one outlet opening which in the closed position of the valve body is closed by the valve seat, and which at its upper part is connected to the measuring vessel through at least one hole in the chamber wall, and (b) an overflow chamber having at its lower end a duct which is directly connected to the liquid outlet of the measuring vessel and which at its top end is connected to the measuring vessel via a hole in the chamber wall, the holes connecting the reception chamber to the measuring vessel and the measuring vessel to the overflow chamber being placed on the same side of the hollow valve body.

When the hollow valve body is in its closed position the outlet opening of the reception chamber is closed by the valve seat as mentioned above and consequently the liquid introduced into the reception chamber gradually fills said chamber which is preferably provided with graduations indicating the amount of liquid contained therein.

When the liquid levels with the hole in the chamber wall the introduction of additional liquid will cause overflow of liquid into the measuring vessel proper, which measuring vessel is thus gradually filled. When the liquid surface in the measuring vessel has reached the level of the hole in the overflow chamber the introduction of additional liquid will cause overflow into the overflow chamber and through the duct being in communication with the liquid outlet into the liquid collection bag.

When emptying the inlet chamber and the measuring vessel the valve body is lifted from the valve seat. This causes the liquid in the reception chamber to flow out through the outlet opening at the lower end of the chamber and allows the flow of liquid to pass from the measuring vessel to the liquid outlet and from here into the liquid collection bag.

Since the holes in the above-mentioned valve body are placed on the same side of the hollow valve body the apparatus may be positioned in an inclined position or horizontally provided said holes face upwards without causing unintentional overflow of liquid from reception chamber to measuring vessel and overflow chamber.

The upper end of the hollow valve body may be provided with a radially extending protrusion placed in a helical groove on the inside of a surrounding part of the measuring vessel. This construction permits a controlled vertical displacement of the hollow valve body above the valve seat by turning of the valve body.

To facilitate turning of the hollow valve body said valve body is preferably provided with a radially extending wing at its top end.

According to an embodiment of the invention one or several protrusions touching the outside of the valve body and serving as a guide therefor may be located on the inside of the measuring vessel.

In order to provide a liquid tight connection between the valve body and the valve seat in the closed position of the valve the valve body is preferably provided with two annular grooves for O-rings in the area where the valve body is in contact with the valve seat.

A similar annular groove with an O-ring is preferably provided at the upper end of the valve body. The latter sealing arrangement prevents outflow of liquid from the measuring vessel at the upper end of same.

The upper end of the valve body is preferably closed by a cover and the cover preferably comprises a liquid inlet comprising a tubular member having an inclined end surface covered by a valve flap preventing back flow of liquid. Preferably the cover also comprises an opening which is covered by an air permeable and liquid proof filter which permits an outflow of air but not of liquid as the reception chamber and the measuring vessel is filled with liquid and which permits an inflow of air when liquid is transferred to the collection bag.

The invention will now be described more in detail with reference to the accompanying drawings wherein
- Figure 1: shows a front view of a preferred embodiment of the apparatus of the invention,
- Figure 2: is a vertical sectional view of the lower part of the valve body shown in figure 1,
- Figure 3: is a side view of the valve body of Figure 2,
- Figure 4: is a sectional view along the line III-III through the valve body of Figure 3,
- Figure 5: is a front view of the back wall of the measuring vessel of the apparatus of Figure 1,
- Figure 6: shows the upper part of the valve body of Figure 1 seen from the bottom, and
- Figure 7: shows the upper part of the valve body illustrated in fig. 6 in sectional view.

In the drawing 1 designates a measuring vessel in which a hollow valve body 2 is placed which is axially displacable by rotation and which at its lower end is in contact with a valve seat 3 and is closed at the top with a cover 4. At opposite sides of the measuring vessel 1 there are provided protrusions 5 and 6 for fixing the ends of suspension straps (not shown).

The measuring vessel 1 also comprises a pair of carrier arms 7 ending in double hooks for insertion into holes 9 at the top end of a liquid collection bag 10 having a liquid inlet 11. At its lower end the liquid collection bag 10 is provided with a liquid outlet 12 in which a pipe 13 is positioned provided with a valve 14.

A wing 15 is attached to the cover 4 of the valve body 2, which cover will be described more in detail in the following.

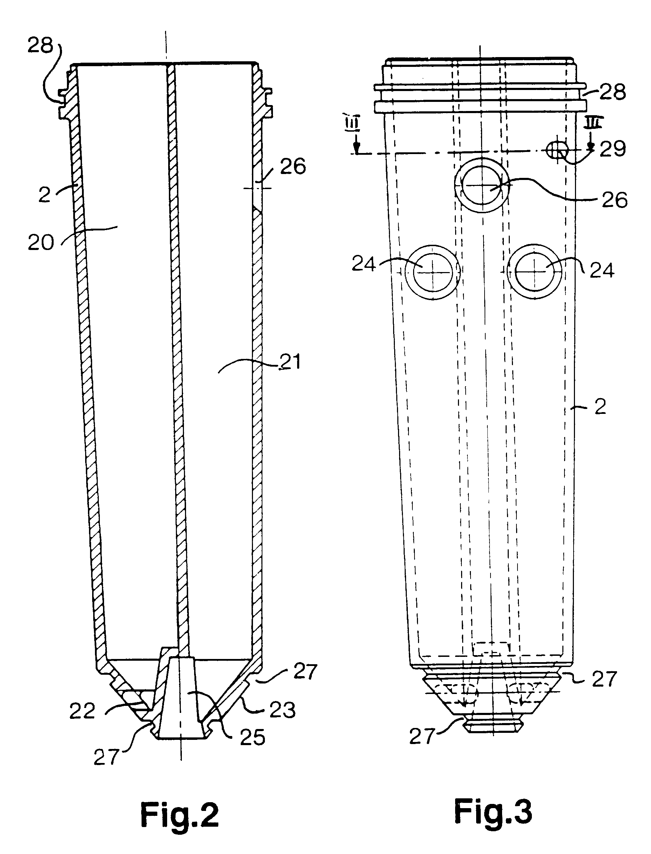

As it will appear from Figures 2-4 the valve body is constructed with two chambers; a reception chamber 20 and an overflow chamber 21.

At its lower end the reception chamber 20 is provided with two holes 22 ending in a valve surface 23 which contacts the valve seat 3 in its closed position thus preventing liquid outflow. At the upper end of the reception chamber 20 two holes 24 are provided in the wall separating the chamber 20 from the surrounding measuring vessel 1.

At its lower end the overflow chamber 21 is provided with a duct 25 through which said overflow chamber is in direct contact with the liquid outlet of the measuring vessel 1. At the top end of the overflow chamber 21 a hole 26 is provided in the valve wall.

Two annular-shaped grooves 27 each comprising an O-ring (not shown) are provided on the outside of the valve body 2 at the lower end thereof.

Similarly, an annular groove 28 comprising an O-ring is provided.

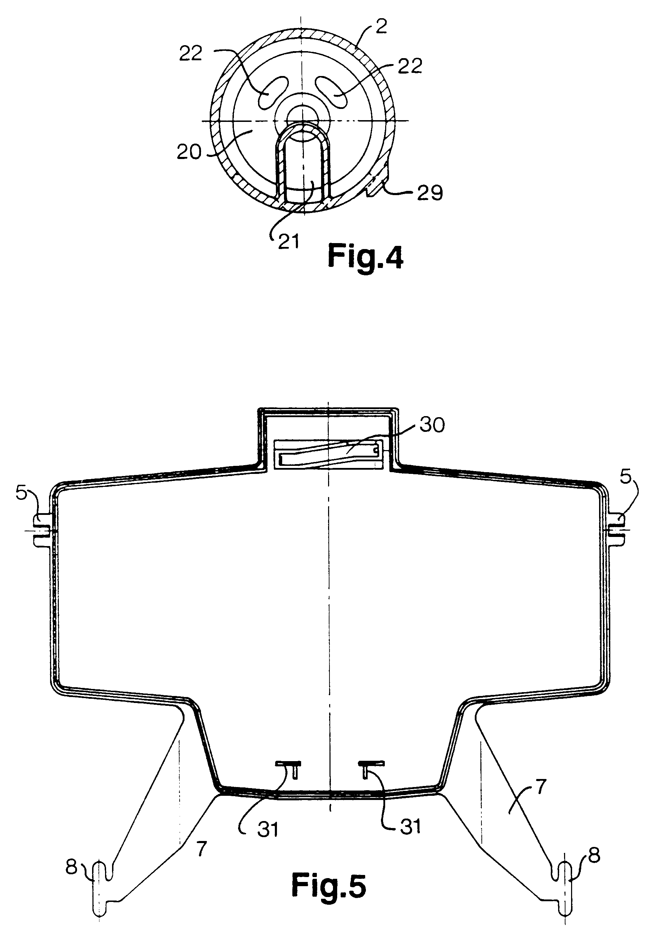

The outside of the valve body 2 is also provided with a projection 29 which is inserted in a helical groove 30 formed on the inside of the back wall of the measuring vessel 1, cf. Figure 5.

As will also appear from Fig. 5 the rear wall comprises two protrusions 31 serving as a guide for the lower part of the valve body 2.

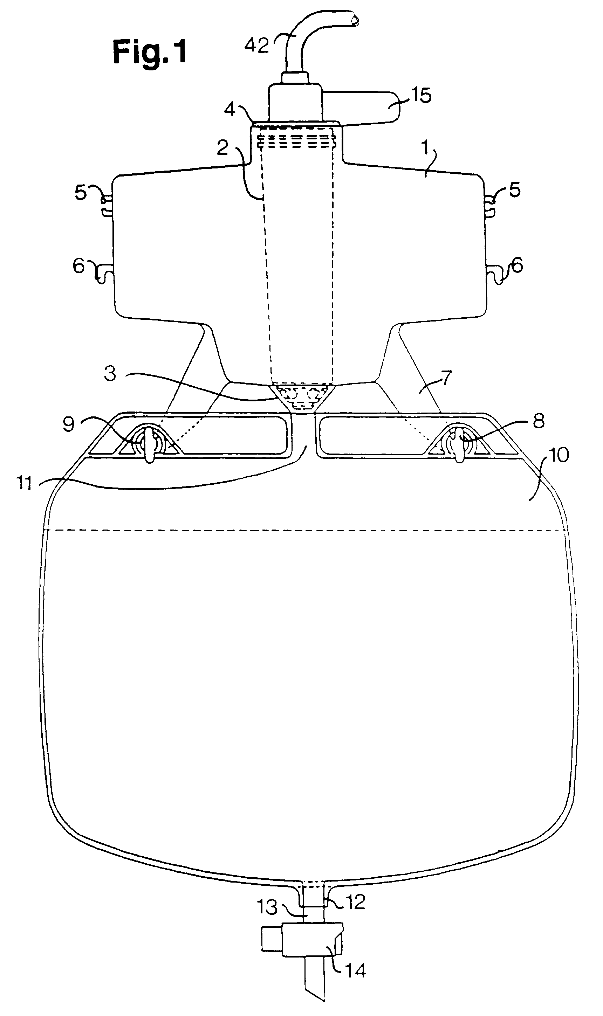

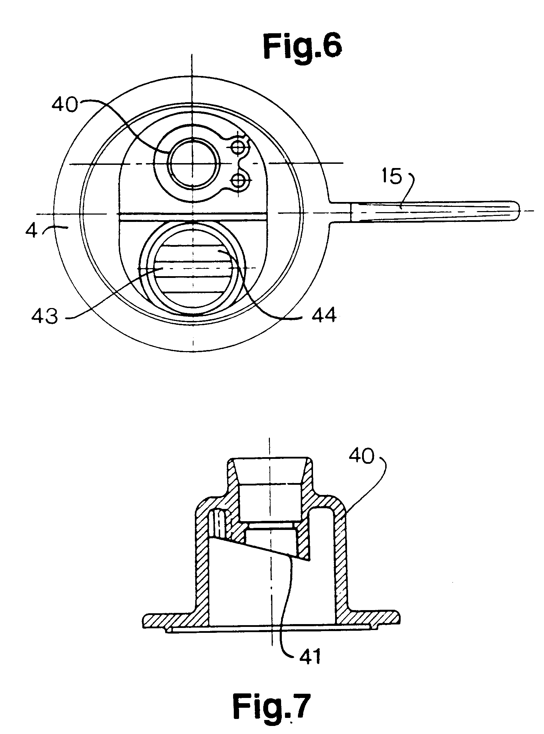

As will appear from Figures 6 and 7 the cover 4 of the hollow valve body is provided with two openings one being a tubular member 40 ending in an inclined surface 41 covered by a rubber flap (not shown) permitting the introduction of liquid and preventing the back flow of same. A catheter tube 42 is attached (cf. Figure 1) to said tubular member.

Next to the tubular member 40 a ventilation opening 43 is provided which is partially covered by support ribs 44 supporting an air filter (not shown).

The cover 4 is preferably welded onto the valve body 2.

The shown apparatus operates as follows:
The liquid introduced through the catheter tube 42 passes down into the reception chamber 20 inside the valve body 2 via the tubular member 40. In its initial (closed) position the valve surface 23 of the valve body 2 is in contact with the valve seat 3 and consequently no liquid is allowed to pass through the holes 22. Therefore, the liquid level in the reception chamber will rise and using a suitable measurement scale, e.g. in the form of graduation marks on the outside of the valve body 2 and a transparent construction material, the volume of the collected liquid at a given time may be read.

When the liquid surface has reached the level of the holes 24 the introduction of additional liquid starts filling of the measuring vessel. The vessel 1 may also be graduated so as to allow for the amount of liquid contained therein to be read off at a given time.

If additional amounts of liquid are introduced the measuring vessel 1 is also filled and through the hole 26 an overflow of liquid from the measuring vessel 1 into the overflow chamber 21 may occur. From here the liquid passes via the duct 25 down through the liquid outlet at 3 and into the liquid collection bag 10.

At this time or at any earlier desired time the reception chamber 20 as well as the measuring vessel 1 may be emptied by clockwise rotation of the wing 15. Such rotation causes the valve body 2 to be rotated and because the projection 29 is located in the groove 30 the valve body is lifted upwards in connection with the rotation thus removing the valve surface 23 from the valve seat 3. Hence it is rendered possible to empty the reception chamber 20 quickly through the holes 22 and the measuring vessel 1 through the space between the valve surface 23 and the valve seat 3 as the liquid flows down into the liquid collection bag 10 through the liquid outlet 11.

The generation of a superatmospheric or subatmospheric pressure in chambers 20 and 21 as well as in the measuring chamber 1 during liquid inflow and outflow, respectively, is avoided due to an air flow being allowed through the filter provided in the ventilation opening 43.

## Claims

1. An apparatus for the collection and measuring of body liquids, said apparatus comprising a measuring vessel (1) having at its top end a liquid inlet (40) and at its lower end a liquid outlet provided with a valve (2,3) and a liquid collection bag (10) connected to the liquid outlet and suspended from the measuring vessel (1), **characterized** in that a hollow valve body (2) is placed in and vertically displaceable within the measuring vessel (1), and that the liquid outlet has the shape of a valve seat (3) for the hollow valve body (2), the interior of the valve body being divided into a reception chamber (20) and an overflow chamber (21); said reception chamber (20) being connected to the liquid inlet (40) and having at the lower end of the valve body (2) at least one outlet opening (22) which in the closed position of the valve body (2) is closed by the valve seat (3) and which at its upper part is connected to the measuring vessel through at least one hole (24) in the chamber wall; and said overflow chamber (21) having at its lower end a duct (25) which is directly connected to the liquid outlet of the measuring vessel (1) and which at its top end is connected to the measuring vessel (1) via a hole (26) in the chamber wall, the holes connecting the reception chamber (20) to the measuring vessel (1) and the measuring vessel (1) to the overflow chamber (21) being placed on the same side of the hollow valve body (2).

2. An apparatus according to claim 1, **characterized** in that the valve body (2) has at its upper end a protrusion (29) extending radially from the outside of the valve body (2) which protrusion (29) is placed in a helical groove (30) on the inside of a surrounding part of the measuring vessel (1) so as to cause a vertical displacement of the valve body (2) by rotation of the valve body (2).

3. An apparatus according to claim 2, **characterized** in that the valve body (2) is provided with a radially extending wing (15) at its upper end.

4. An apparatus according to claim 1, **characterized** in that the inside of the measuring vessel (1) comprises one or several protrusions (31) which are in contact with the outside of the valve body (2) and serve as a guide for same.

5. An apparatus according to any one of the preceding claims, **characterized** in that two annular grooves (27) for O-rings are provided in that zone of the valve body (2) which is in contact with the valve seat (3) in its closed position.

6. An apparatus according to any one of the preceding claims, **characterized** in that the valve body (2) is closed at the top by a cover (4).

7. An apparatus according to claim 6, **characterized** in that in the cover (4) comprises a liquid inlet (40) in the form of a tubular member having an inclined end surface covered by a valve flap.

8. An apparatus according to claim 6, **characterized** in that in the cover (4) comprises an opening (43) which is covered by an air permeable and liquid tight filter.

## Patentansprüche

1. Vorrichtung zum Sammeln und Messen von Körperflüssigkeiten, umfassend ein Meßgefäß (1), das an seinem oberen Ende einen Flüssigkeitseinlaß (40) und an seinem unteren Ende einen mit einem Ventil (2, 3) versehenen Flüssigkeitsauslaß aufweist, und einen Flüssigkeitssammelbeutel (10), der mit dem Flüssigkeitsauslaß verbunden und an dem Meßgefäß (1) aufgehängt ist, dadurch **gekennzeichnet**, daß ein hohler Ventilkörper (2) in dem Meßgefäß (1) vertikal verlagerbar angeordnet ist und daß der Flüssigkeitsauslaß die Form eines Ventilsitzes (3) für den hohlen Ventilkörper (2) hat, wobei das Innere des Ventilkörpers in eine Aufnahmekammer (20) und eine Überlaufkammer (21) unterteilt ist; wobei die Aufnahmekammer (20) mit dem Flüssigkeitseinlaß (40) verbunden ist und am unteren Ende des Ventilkörpers (2) mindestens eine Auslaßöffnung (22) aufweist, die in der Schließstellung des Ventilkörpers (2) durch den Ventilsitz (3) verschlossen ist und die an ihrem oberen Teil über mindestens eine Öffnung (24) in der Kammerwand mit dem Meßbehälter verbunden ist; und wobei die Überlaufkammer (21) an ihrem unteren Ende einen Kanal (25) aufweist, der mit dem Flüssigkeitsauslaß des Meßgefäßes (1) unmittelbar verbunden ist und der an seinem oberen Ende über eine Öffnung (26) in der Kammerwand mit dem Meßgefäß (1) verbunden ist, wobei die die Aufnahmekammer (20) mit dem Meßgefäß (1) und das Meßgefäß (1) mit der Überlaufkammer (21) verbindenden Öffnungen auf der gleichen Seite des hohlen Ventilkörpers (2) angeordnet sind.

2. Vorrichtung nach Anspruch 1, dadurch **gekennzeichnet**, daß der Ventilkörper (2) an seinem oberen Ende einen Vorsprung (29) hat, der sich von der Außenseite des Ventilkörpers (2) radial erstreckt, wobei der Vorsprung (29) in eine Gewindenut (30) an der Innenseite eines Umfangsteils des Meßgefäßes (1) eingreift, um bei einer Verdrehung des Ventilkörpers (2) eine vertikale Verlagerung des Ventilkörpers (2) zu bewirken.

3. Vorrichtung nach Anspruch 2, dadurch **gekennzeichnet**, daß der Ventilkörper (2) an seinem oberen Ende mit einer radial abstehenden Nase (15) versehen ist.

4. Vorrichtung nach Anspruch 1, dadurch **gekennzeichnet**, daß die Innenseite des Meßgefäßes (1) einen oder mehrere Vorsprünge (31) aufweist, die mit der Außenseite des Ventilkörpers (2) in Berührung stehen und als Führung für diesen dienen.

5. Vorrichtung nach einem der vorhergehenden Ansprüche, dadurch **gekennzeichnet**, daß zwei Ringnuten (27) für O-Ringe in derjenigen Zone des Ventilkörpers (2) vorgesehen sind, die in dessen Schließstellung mit dem Ventilsitz (3) in Berührung steht.

6. Vorrichtung nach einem der vorhergehenden Ansprüche, dadurch **gekennzeichnet**, daß der Ventilkörper (2) an seiner Oberseite mit einem Deckel (4) verschlossen ist.

7. Vorrichtung nach Anspruch 6, dadurch **gekennzeichnet**, daß der Deckel (4) einen Flüssigkeitseinlaß (40) in Form eines rohrförmigen Teils umfaßt, der eine von einer Ventilklappe bedeckte geneigte Endfläche hat.

8. Vorrichtung nach Anspruch 6, dadurch **gekennzeichnet**, daß der Deckel (4) eine Öffnung (43) umfaßt, die mit einem luftdurchlässigen und flüssigkeitsdichten Filter bedeckt ist.

## Revendications

1. Un appareil pour recueillir et mesurer des liquides corporels, ledit appareil comportant un récipient de mesure (1) présentant à son extrémité supérieure une entrée de liquide (40) et à son extrémité inférieure une sortie de liquide munie d'un clapet (2,3), et un sac (10) de recueil de liquide relié à la sortie de liquide et suspendu au récipient de mesure (1), caractérisé en ce qu'un corps creux (2) de clapet est placé de manière déplaçable verticalement à l'intérieur du récipient de mesure (1), et en ce que la sortie de liquide présente la forme d'un siège de clapet (3) pour le corps de clapet (2), l'intérieur du corps de clapet étant divisé en une chambre de réception (20) et une chambre de trop-plein (21), ladite chambre de réception (20) étant reliée à la sortie de liquide (40) et présentant, à l'extrémité inférieure du corps de clapet (2), au moins une ouverture de sortie (22) qui, pour la position fermée du corps de clapet (2), est fermée par le siège de clapet (3) et qui, à sa partie supérieure, est reliée au récipient de mesure à travers au moins un orifice (24) de la paroi de chambre, et ladite chambre de trop-plein (21) présentant à son extrémité inférieure une conduite (25) qui est reliée directement à la sortie de liquide du récipient de mesure (1) et qui est reliée à son extrémité supérieure au récipient de mesure (1) par l'intermédiaire d'un orifice (26) de la paroi de chambre, les orifices reliant la chambre de réception (20) au récipient de mesure (1) et le récipient de mesure (1) à la chambre de trop-plein (21) étant placés du même côté du corps creux de clapet (2).

2. Un appareil selon la revendication 1, caractérisé en ce que le corps de clapet (2) présente à son extrémité supérieure une saillie (29) s'étendant radialement à partir de l'extérieur du corps de clapet (2), cette saillie (29) étant placée dans une gorge hélicoïdale (30) du côté intérieur d'une partie entourante du récipient de mesure (1) de manière à provoquer un déplacement vertical du corps de clapet (2) par la rotation du corps de clapet (2).

3. Un appareil selon la revendication 2, caractérisé en ce que le corps de clapet (2) est muni à son extrémité supérieure d'une ailette (15) s'étendant radialement.

4. Un appareil selon la revendication 1, caractérisé en ce que l'intérieur du récipient de mesure (1) comporte une ou plusieurs saillies (31) qui sont en contact avec l'extérieur du corps de clapet (2) et qui servent de guide pour celui-ci.

5. Un appareil selon l'une quelconque des revendications précédentes, caractérisé en ce que deux gorges annulaires (27)pour des joints toriques sont prévues dans la zone du corps de vanne (2) qui est en contact avec le siège de clapet (3) dans sa position fermée.

6. Un appareil selon l'une quelconque des revendications précédentes, caractérisé en ce que le corps de clapet (2) est fermé en haut par un couvercle (4).

7. Un appareil selon la revendication 6, caractérisé en ce que le couvercle (4) comporte une entrée de liquide (40) sous la forme d'un élément tubulaire présentant une surface extrême inclinée recouverte par un volet de clapet.

8. Un appareil selon la revendication 6, caractérisé en ce que le couvercle (4) comporte une ouverture (43) qui est recouverte par un filtre perméable à l'air et imperméable aux liquides.
